# EUROPÄISCHE PATENTSCHRIFT

(11) **EP 0 991 358 B1**
(45) Veröffentlichungstag und Bekanntmachung des Hinweises auf die Patenterteilung: **11.08.2004**
(21) Anmeldenummer: 98936376.7
(22) Anmeldetag: 19.06.1998
(51) Int. Cl.: A61B 10/00, B01L 3/00

(54) **VORRICHTUNG ZUR AUFNAHME UND UNTERSUCHUNG VON PROBEN**
DEVICE FOR TAKING AND EXAMINING SAMPLES
DISPOSITIF POUR LE PRELEVEMENT ET L'EXAMEN D'ECHANTILLONS

(30) Priorität: 20.06.1997 DE 19726268
(43) Veröffentlichungstag der Anmeldung: 12.04.2000
(73) Patentinhaber: DakoCytomation Denmark A/S, 2600 Glostrup (DK)
(72) Erfinder: SANNER, Stefan, D-82110 Germering (DE)
(74) Vertreter: Eisenführ, Speiser & Partner
(86) Internationale Anmeldenummer: PCT/EP1998/003764
(87) Internationale Veröffentlichungsnummer: WO 1998/058587

(56) Entgegenhaltungen:
- EP-A- 0 520 408
- DE-A- 4 117 635

## Beschreibung

Die vorliegende Erfindung betrifft eine Vorrichtung zur Aufnahme und Untersuchung von Proben, insbesondere zur integrierten Probensammlung und Untersuchung mittels Testverfahren, insbesondere immunologischer, chemischer oder biochemischer Art.

In der DE 41 17 635 C2 wird eine Mehrzweck-Untersuchungsvorrichtung für Stuhlproben beschrieben. Diese umfaßt ein Gehäuse mit einem Hohlraum und einer seitlich angeordneten Röhre. Mit einem Rührabschnitt wird eine Stuhlprobe aufgenommen und in den Hohlraum eingebracht und dieser mit einer Kappe geschlossen. Ein innerer Anschluß der Röhre wird mit der Stuhlprobe benetzt. Mittels einer Spritze wird ein hochempfindliches Reagenz, wie O-Toluidin oder Tetramethyl-benzhydrin und Wasserstoffperoxid in den Hohlraum eingebracht. Aufgrund einer Farbreaktion wird geprüft, ob die genommene Stuhlprobe Blutspuren enthält. Für einen weiteren Test wird durch eine Öffnung am freien Ende der Röhre eine Testreagenz geringerer Empfindlichkeit wie Guajac-Harz oder Lignumvitae-Harz und Wasserstoffperoxid eingefüllt. Durch Beobachtung einer entsprechenden Farbreaktion wird untersucht, ob die Stuhlprobe Blutspuren enthält. Diese Untersuchungsvorrichtung hat einen komplizierten Aufbau mit einer Vielzahl von Einzelteilen und ist schwierig zu handhaben.

In der EP-0 327 144 A2 wird ein Probenaufnahmegefäß und ein Verfahren zur Verarbeitung von pastösem Probenmaterial beschrieben. An einem Gefäßdeckel ist über einen Stiel ein Probenaufnahmebecher angeordnet, mit dem eine Probe aufgenommen und in das Aufnahmegefäß eingebracht wird. In dem Aufnahmegefäß ist eine Flüssigkeit vorhanden, die zur Aufschlämmung der Stuhlprobe dient. Beim Einsetzen des Probenbechers und dem anschließenden Aufschrauben des Gefäßdeckels wird die Stuhlprobe in der Flüssigkeit verteilt. Nach dem Transport in ein Labor wird der Gefäßdeckel abgenommen und eine zweite Flüssigkeit, insbesondere organisches Lösungsmittel (Ether oder Ethylacetat) oder Farbmittel (z.B. lugolsche Lösung) zugegeben. Zur anschließenden Filtrierung wird ein separater Filterkörper aufgeschraubt und durch Schütteln die filtrierte Suspension im Filtratgefäß erhalten. Die filtrierte Suspension kann anschließend untersucht werden. Diese Vorrichtung besteht aus zahlreichen einzelnen Bauelementen und ist schwierig zu handhaben. Insbesondere besteht die Gefahr, daß die Flüssigkeit beim Handhaben des Gefäßes vergossen wird.

Die US-A-4 978 504 beschreibt eine Testeinheit zur Aufnahme und Analyse von Proben. Die Testeinheit besteht aus einem Halter mit einem an einem Ende eines Stäbchens angeordneten Aufnahmeschwamm, mit dem eine Probe aufgenommen werden kann. Der Halter weist einen Hohlraum auf, in dem eine zerstörbare Ampulle mit einer Reaktionsflüssigkeit vorgesehen ist. Nach Aufnahme einer Probe wird das Stäbchen in ein rohrförmiges Gehäuse eingeführt und die Halteeinrichtung aufgesteckt. Anschließend wird die Ampulle durch Eindrücken der Halteeinrichtung zerstört und die Reaktionsflüssigkeit fließt in das Gehäuse und kommt mit der Probe in Berührung. Anschließend wird die Mischung in einem Gefäß aufgefangen oder auf einem Träger aufgetropft und mit separaten Vorrichtungen untersucht.

In der EP-A-0 520 408, die als nächst Kommender Stand der Technik angesehen wird, wird eine Vorrichtung beschrieben, die insbesondere zur Untersuchung von Speichelproben geeignet ist. Diese Testeinheit besteht aus einem zylindrischen Behälter, in dem eine Flüssigkeit enthalten ist und einer als Kolben mit einem daran angeordneten Schwamm zur Aufnahme einer Speichelprobe ausgebildeten Aufnahmeeinrichtung. Nach Aufnahme einer Probe wird die Aufnahmeeinrichtung in das Gehäuse eingeschoben und der Aufnahmeschwamm kommt mit der Flüssigkeit in Berührung. Die Testeinrichtung weist außerdem einen Auffangbehälter auf, der auf das andere Ende des Gehäuses aufsetzbar ist und gegenüber dem Gehäuse verschiebbar ist. Im Innern des Aufnahmegefäßes ist ein Dorn angeordnet, der einer Öffnung am anderen Ende des Gehäuses gegenüberliegt, die von einer Folie verschlossen ist. In der aufgeschobenen Position des Aufnahmegefäßes wird mit Hilfe des Dorns die Folie zerstört und damit die Öffnung freigegeben. Durch vollständiges Einschieben der kolbenartigen Aufnahmeeinrichtung wird die Flüssigkeit vom Behälter in den Auffangbehälter gepreßt und gleichzeitig die schwammartige Aufnahmeeinrichtung ausgedrückt. In der Aufnahmeeinrichtung befindet sich eine Reaktionssubstanz, die mit der Probe und der Flüssigkeit reagiert, wobei diese Flüssigkeit anschließend mit einer getrennten Vorrichtung untersucht wird. Die US-A-5 393 496 beschreibt eine ähnliche Testeinrichtung.

Der vorliegenden Erfindung liegt die Aufgabe zugrunde, eine Vorrichtung zur Aufnahme und Untersuchung von Proben bereitzustellen, die einfach aufgebaut ist und leicht zu handhaben ist.

Diese Aufgabe wird mit den Merkmalen der unabhängigen Patentansprüche gelöst. Vorteilhafte Aus-bzw. Weiterbildungen der Erfindung sind in den abhängigen Ansprüchen definiert. Die erfindungsgemäße Vorrichtung hat insbesondere den Vorteil, daß Proben einfach aufgenommen werden können, die Aufnahmeeinrichtung mit der Probe sicher in ein Gehäuse einführbar ist und darin mit einer Probenaufarbeitungsflüssigkeit in einfacher Weise vermischbar ist und danach mit einer im Gehäuse angeordneten Testeinrichtung die Untersuchung der Probe durchführbar ist.

Weiterhin hat die erfindungsgemäße Vorrichtung den Vorteil, daß eine Probenaufarbeitungsflüssigkeit in der Patrone sicher verschlossen ist und der Benutzer auch nach Vermischen mit der Probe nicht in Berührung kommt.

Die Erfindung wird nachstehend anhand eines Ausführungsbeispiels mit Bezug auf die Zeichnung näher erläutert. Es zeigen:
- Fig. 1: eine Prinzipskizze des Aufbaus einer erfindungsgemäßen Vorrichtung
- Fig. 2: eine griffseitige Teilansicht einer Ausführungsform einer erfindungsgemäßen Aufnahmeeinrichtung,
- Fig. 3: eine probenseitige Teilansicht der Aufnahmeeinrichtung von Fig. 2,
- Fig. 4: eine Prinzipskizze der erfindungsgemäßen Vorrichtung und
- Fig. 5: eine vergrößerte perspektivische Teilansicht der Vorrichtung von Fig. 4.

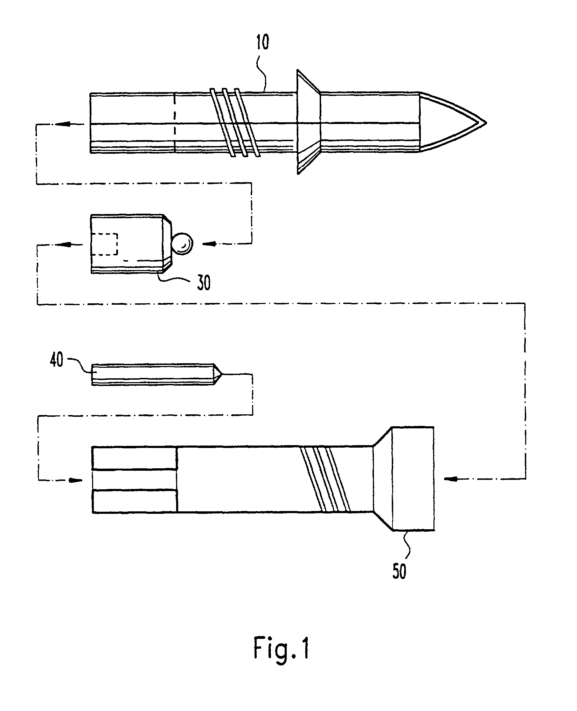

Die in Fig. 1 gezeigte Vorrichtung umfaßt eine Aufnahmeeinrichtung 10, eine Patrone 30, eine Testeinrichtung 40 und ein Gehäuse 50. Wie durch die gestrichelten Linien angedeutet, wird zunächst die Patrone 30 in das rohrförmige Gehäuse 50 eingesetzt und anschließend die Aufnahmeeinrichtung 10 von der gleichen Seite in das Gehäuse 50 eingeführt. An der gegenüberliegenden Seite des Gehäuses 50 wird eine Testeinrichtung 40 eingeführt. Vorzugsweise ist die Patrone 30 und/oder die Testeinrichtung 40 in dem Gehäuse 50 verschiebbar und gegen ein Herausfallen gesichert.

Vorzugsweise wird die Vorrichtung in der zuvor beschriebenen Weise zusammengesetzt und einem Benutzer zur Aufnahme einer Probe und Untersuchung bereitgestellt. Diese Anordnung stellt einen Testkit dar, der in einfacher Weise zu handhaben ist und auch von Laien, insbesondere älteren Personen einfach zu bedienen ist.

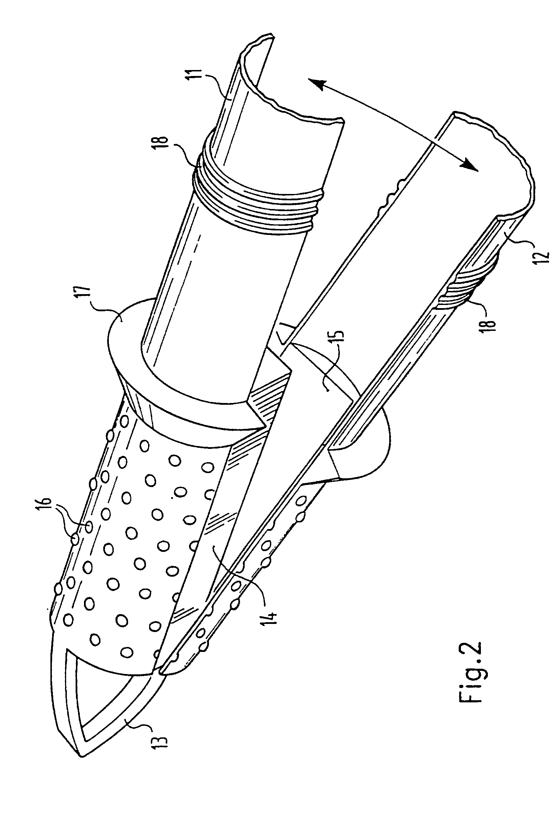

Wie in Fig. 2 gezeigt, besteht die Aufnahmeeinrichtung 10 aus zwei Halbröhren 11, 12, die im geschlossenen Zustand eine Röhre (zylinderförmiger Hohlraum) bilden. Die beiden Halbröhren sind an einem Ende (Griffseite) durch einen elastischen Steg 13 miteinander verbunden, dessen Spannung die beiden Hälften auseinanderdrückt, so daß diese im entspannten Zustand die Form eines "V" ergeben. Bei einer alternativen Ausführungsform können die beiden Halbröhren gelenkig verbunden sein, wobei vorzugsweise die Gelenkverbindung ein Filmscharnier aufweist. Beispielsweise könnte das Filmscharnier innerhalb einer Stegverbindung zwischen den Halbröhren angeordnet sein. Vorzugsweise ist die Stegverbindung ähnlich gestaltet wie der Steg 13 in Fig. 2 und das Filmscharnier ist im Bereich des spitzen Endes angeordnet. Bei dieser Ausführungsform sind beide Halbröhren frei gegeneinander verschwenkbar. Falls eine Federwirkung gewünscht ist, können zusätzliche Federmittel vorgesehen werden. An der Innenseite der einen Halbröhre 11 ist ein dreieckförmiger länglicher Vorsprung 14 angeordnet, der mit einer an der anderen Halbröhre 12 ausgebildeten entsprechenden Gegennut 15 in Eingriff bringbar ist. Der Vorsprung 14 mit der Gegennut 15 bilden eine Führung, die beim Schließen der Aufnahmeeinrichtung ein seitliches Ausscheren der beiden Halbröhren verhindern. Auf der Außenseite der Halbröhren ist am griffseitigen Ende eine Grifffläche mit Noppen 16 versehen, die ein Abrutschen beim Hantieren verhindern. Die Grifffläche wird zur Probenseite hin abgeschlossen durch einen trichterförmigen, rundum laufenden Rand 17, der einen Kontakt mit überschüssigem Probenmaterial vermeiden hilft. Probenseitig des umlaufenden Rands 17 ist an der Außenseite der aus den beiden Halbröhren gebildeten Röhre ein Steilgewinde 18 ausgebildet.

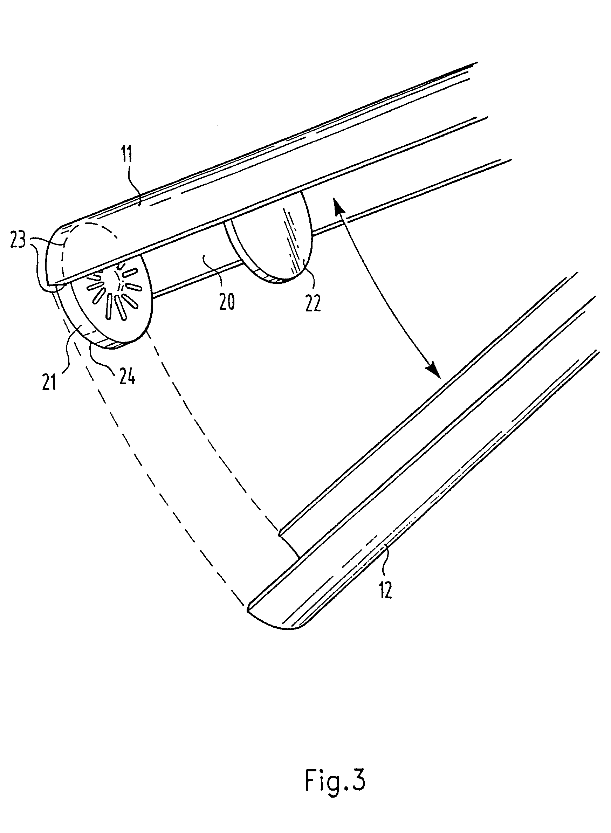

Wie in Fig. 3 dargestellt, sind am probenseitigen Ende der Aufnahmeeinrichtung 10 an der einen Halbröhre 11 eine erste Scheibe 21 und im Abstand davon eine zweite Scheibe 22 angeordnet. Beim Schließen der Aufnahmeeinrichtung, d.h. beim Aufeinanderbringen der beiden Halbröhren 11, 12, bilden diese beiden Scheiben eine Probenkammer 20. Aus dem Abstand a der beiden Scheiben zueinander und dem Radius r der Halbröhren ergibt sich das aufzunehmende Probenvolumen V wie folgt V = Ð · r² · a. Die Länge der Halbröhren 11, 12 ist dabei so dimensioniert, daß zwischen der griffseitigen Scheibe 22 und der vorstehend beschriebenen Führung 14, 15 ein ausreichender Raum für eventuell überschüssiges Probenmaterial gebildet wird.

Die probenseitige Abschlußscheibe 21 ist an der Innenseite der Halbröhre 11 derart befestigt, daß die Verbindung den bei der Probennahme auftretenden Kräften widersteht, jedoch beim Einführen in das Gehäuse die Scheibe 21 durch einen in dem Gehäuse auftretenden Widerstand an einer oder mehreren Sollbruchstellen 23 herausgebrochen wird. An der Außenkante dieser Scheibe 21 ist ein konusförmiger Ring 24 aufgesetzt, dessen Funktion nachstehend näher erläutert wird. In der Mitte der Scheibe 21 ist eine Mulde 25 ausgebildet. Die durch diese Mulde gebildete Fläche entspricht einer Öffnung eines Kegelstumpfes an der Patrone. Die Scheibe 21 ist außerhalb der Mulde 25 so durchbrochen, daß ein Sieb 26 zum mechanischen Aufschluß der Probe gebildet wird. Alternativ zu der Mulde 25 kann ein Steg vorgesehen sein, der nachstehend näher beschrieben wird.

Die griffseitige Scheibe 22 ist fest angebracht, so daß sie allen bei der Probennahme und Probenverarbeitung auftretenden Kräften widersteht.

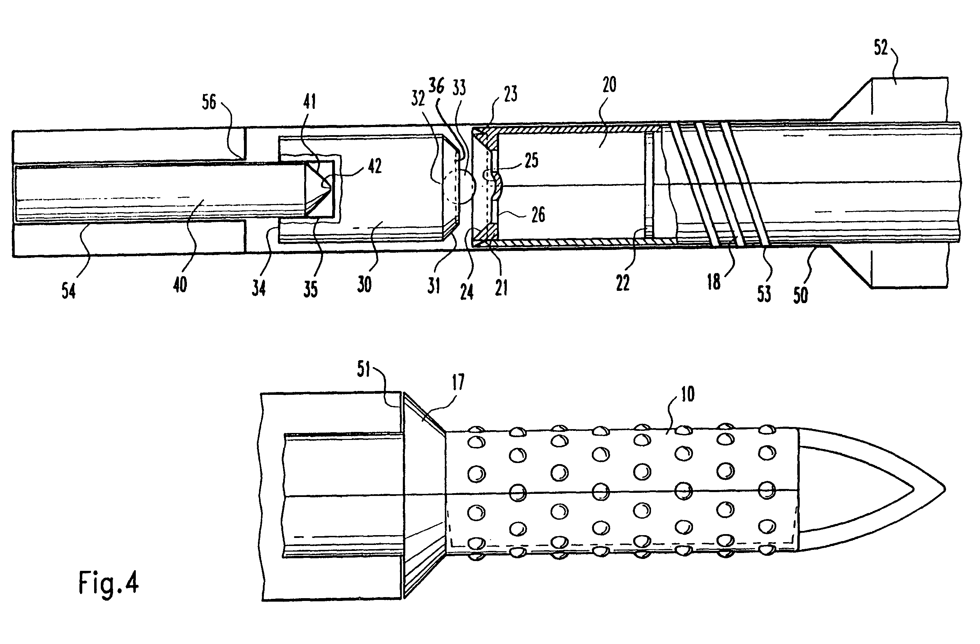

Mit weiterer Bezugnahme auf Fig. 4 wird nun zunächst die Patrone 30 näher beschrieben. In der Patrone 30 ist eine Flüssigkeit enthalten, die beispielsweise zur Verdünnung und zum chemischen Aufschluß der Probe dient. Die Größe des Innenraums der Patrone 30 und die Flüssigkeitsmenge M sind so gewählt, daß die Patrone das Probenvolumen V zusätzlich zur vorhandenen Flüssigkeit aufnehmen kann. Die Flüssigkeitsmenge M wird in einem genau dosierten Verhältnis zum vorgegebenen Probenvolumen bereitgestellt. Die Patrone besitzt die Form eines Zylinders. An dem zur Aufnahmeeinrichtung weisenden Ende verjüngt sich der Zylinder zu einem Kegelstumpf 31 dergestalt, daß sich das Sieb 26 an der Aufnahmeeinrichtung mit dem daran befindlichen konusförmigen Ring 24 auf der Patrone selbst zentriert. Dieser konusförmige Sitz dient vorzugsweise gleichzeitig als Dichtung zwischen der Aufnahmeeinrichtung und der Patrone. Am Ende des Kegelstumpfes der Patrone ist eine kreisförmige Vertiefung 36 mit einer mittig angeordneten Öffnung 32 ausgebildet. Die Öffnung wird von einer darin angeordneten Metallkugel 33 verschlossen. Die Metallkugel 33 wird beispielsweise durch Aufschrumpfen in der Öffnung befestigt. Die Größe der Metallkugel 33 ist dabei geringfügig größer als die Öffnung 32 am kegelstumpfförmigen Ende, so daß die Kugel beim Einführen der Aufnahmeeinrichtung in das Gehäuse in die Patrone 30 gedrückt werden kann. Zum Eindrücken der Kugel 33 dient alternativ die Mulde 25 oder ein an dem Sieb angeordneter Steg. Ein Steg ist insbesondere von Vorteil, wenn die Kugel in der Deckfläche der Patrone vertieft angeordnet ist. Die Dimensionen werden so gewählt, daß vorzugsweise die Konusflächen dichtend aneinander liegen, sobald die Kugel vollständig in die Patrone eingedrückt ist.

Das gegenüberliegende Ende der zylinderförmigen Patrone 30 ist durch einen Boden 34 verschlossen, in dessen Mitte sich eine in die Patrone 30 hineinragende, ebenfalls zylinderförmige Einbuchtung 35 befindet. Diese Einbuchtung 35 dient zur Aufnahme der Testeinrichtung 40. Die Einbuchtung und die Testeinrichtung sind so ausgebildet, daß die Wandung der Einbuchtung 35 durchstoßen werden kann und damit eine Verbindung des Patroneninhalts mit einem in der Testeinrichtung vorhandenen Teststreifen hergestellt werden kann.

Die Testeinrichtung 40 hat die Form eines Zylinders und weist am patronenseitigen Ende eine Verjüngung in Form eines Kegelstumpfs 41 auf. An der Spitze des Kegelstumpfs befindet sich eine Öffnung 42, durch die Probenlösung eintreten kann. In den Kegelstumpf ist ein saugfähiges Material eingebracht, das über Kapillarkräfte die Probenflüssigkeit auf den Teststreifen (nicht gezeigt) transportiert. Die zylinderförmige Testeinrichtung (40) besitzt eine oder mehrere Aussparungen, so daß Farbreaktionen des Teststreifens als Ergebnis des Tests erkennbar sind. Den Abschluß bildet vorzugsweise ein poröses, wasserabweisendes Material, das ein Austreten der Probenflüssigkeit aus der Testeinrichtung verhindert, das Entweichen der von der Probenflüssigkeit verdrängten Luft aber ermöglicht.

Das Gehäuse 50 dient zur Aufnahme, zum Schutz und zur Koordinierung der vorstehend beschriebenen drei Komponenten, der Aufnahmeeinrichtung 10, der Patrone 30 und der Testeinrichtung 40. Es besteht aus einer Röhre, die im Inneren in verschiedene Durchmesser aufgeteilt ist, und im vorderen Teil ein Gewinde 53 aufweist. Das als Einschuböffnung dienende vordere Ende ist in der hier dargestellten Ausführungsform trichterförmig mit einem hohen Rand ausgebildet und hat vorzugsweise einen wesentlich größeren Durchmesser als das probenseitige Ende der Aufnahmeeinrichtung. Das stirnseitige Ende 51 des Gehäuses kommt mit dem trichterförmigen, rund umlaufenden Rand 17 in Berührung und verschließt die Einschuböffnung. Dadurch wird eine Abstreifkammer 52 gebildet, in der außen am probenseitigen Ende der Aufnahmeeinrichtung vorhandenes überschüssiges Probenmaterial abgestriffen und endgelagert wird. Im Anschluß an die Abstreifkammer 52 verjüngt sich der Durchmesser der Röhre, so daß das probenseitige Ende der Aufnahmeeinrichtung verschlossen bleibt und bis zum Eingreifen mit einem in diesem Teil an der Innenseite befindlichen Gewinde 53 verschoben werden kann. Am anderen Teil des Gehäuses ist ein Aufnahmeraum 54 vorgesehen, der in Größe und Form an die Testeinrichtung 40 so angepaßt ist, daß die hülsenförmige Testeinrichtung in ihr gehalten wird, sich aber unter leichtem Druck hin- und herschieben läßt. Im Mittelteil 55 befindet sich die nur durch die Testeinrichtung gehaltene aber sonst frei hängende Patrone 30, die sich zusammen mit der Testeinrichtung 40 verschieben läßt.

Mit der vorstehend beschriebenen Vorrichtung wird ein Test wie folgt durchgeführt.

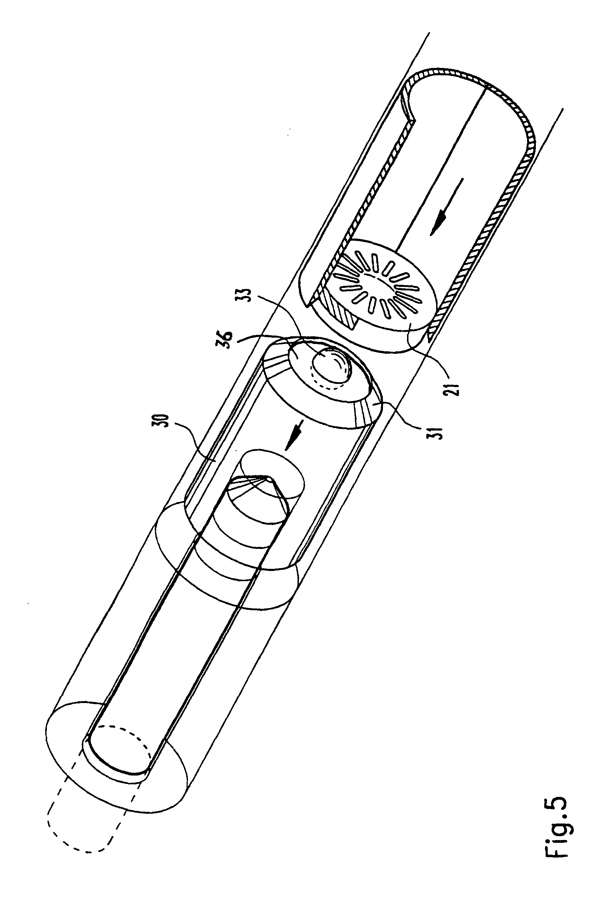

Die Testanordnung ist folgendermaßen montiert. Die Aufnahmeeinrichtung 10 steckt herausnehmbar in dem Gehäuse 50. Die Testeinrichtung 40 und die Patrone 30 sind ineinander gesteckt. Die Kombination aus beiden ist im hinteren Teil des Gehäuses 50 fixiert. Der Benutzer zieht die Aufnahmeeinrichtung aus dem Gehäuse 50. Wenn die Verbindung zwischen den Halbröhren einen elastischen Steg 13 aufweist, öffnet sich die Aufnahmeeinrichtung selbsttätig. Wenn eine Gelenkverbindung vorgesehen ist, z.B. ein Filmscharnier, schwenkt der Benutzer die Halbröhren auseinander. Die beiden Halbröhren 11 und 12 bilden ein "V". Im Bereich der Probenkammer 20 wird nun die Probensubstanz gegriffen und fixiert. Durch Zusammendrücken der Halbröhren schiebt sich die Vorderkante des am vorderen Ende montierten Siebes 26 durch die Probe und wird durch die zweite Halbröhre auf der gegenüberliegenden Seite gehalten. Während des Vorganges des Zusammendrükkens der Halbröhren kommen der dreieckförmige Vorsprung 14 und die Gegennut 15 miteinander in Eingriff, eine Scherwirkung wird unterbunden und die zwei Halbröhren 11, 12 verschließen sich wieder paßgenau zu einer ganzen Röhre. In der Probenkammer 20 ist die Probensubstanz enthalten. In der Probenkammer ist eine genau definierte Menge enthalten, etwa vorhandener Überschuß des Probenmaterials befindet sich in dem hinteren Teil der Aufnahmeeinrichtung, d.h. der Überschußkammer. Die Aufnahmeeinrichtung wird nun in das Gehäuse eingeführt und durch das trichterförmige Auslaufen der Abstreifkammer 52 zur Mitte hin zentriert. Die Aufnahmeeinrichtung wird bis zum Anschlagen des Außengewindes 18 an dem Innengewinde 53 eingeschoben. Das vordere Sieb 26 liegt nun mit seiner halbkugelförmigen Mulde 25 an der Verschlußkugel 33 der Patrone 30 an. Durch das nun beginnende Eindrehen der Aufnahmeeinrichtung in das Gehäuse schiebt sich die durch die Testeinrichtung 40 gehaltene und durch das Sieb 26 an der Aufnahmeeinrichtung geschobene Patrone 30 nach hinten (vgl. Fig. 5). Sie findet dann an der Verjüngungsstelle 56 für die Testeinrichtung einen Anschlag und wird dort fixiert. Durch diesen Vorgang wird die Testeinrichtung ebenfalls nach hinten verschoben und tritt aus dem hinteren Ende des Gehäuses hervor. Dieses nach hinten Hinausschieben der Patronen-Testeinrichtungskombination bis zur tatsächlichen Fixierung verhindert eine vorzeitige ungewollte Aktivierung des Teststreifens. Im weiteren Verlauf der Drehbewegung wird die Verschlußkugel 33 von dem Sieb 26 in die Patrone 30 gedrückt. Das Sieb 26 wird danach auf der Patrone 30 durch den jetzt ineinander greifenden Konus fixiert. Durch den dann entstehenden Druck auf das Sieb 26 wird die Sollbruchstelle 23 getrennt, das lose Sieb 26 wird mittels der Probe auf die Patrone 30 gedrückt. Die Probe wird anschließend durch den Gegendruck der Trennwand 22 zwischen Proben- und Überschußkammer durch das Sieb 26, den von der Vertiefung 36 gebildeten Raum und die Öffnung 32 in die Patrone 30 gepreßt.

Nach Beendigung dieses Vorganges befindet sich die Probe in der Patrone 30, welche durch das aufliegende Sieb 26 und die darauf drückende Trennwand 22 verschlossen wird.

Um die, durch das Sieb 26 schon leicht verquirlte Probe endgültig mit der in der Patrone 30 vorhandenen Flüssigkeit zu vermischen, wird der ganze Testapparat nun geschüttelt. Die im Inneren der Patrone 30 befindliche Verschlußkugel 33 dient nun als Mischkugel, was sich auch durch ein klackerndes Geräusch akustisch bemerkbar macht. Bei einem nachlassenden Geräuschpegel kann der Benutzer davon ausgehen, daß sich die Probe genügend mit der Flüssigkeit vermischt hat und durch ihre höhere Viskosität die Kugel bremst.

Der nächste Schritt besteht nun darin, die aus dem hinteren Ende des Gehäuses ragende Testeinrichtung zu aktivieren. Es funktioniert wie bei einem Kugelschreiber durch einen kurzen Druck auf das Ende der Testeinrichtung. Bei diesem Vorgang wird die Membran in der Rückseite der Patrone 30 vom kegelförmigen Ende 41 der Testeinrichtung durchstoßen. Die Flüssigkeit in der Patrone 30 dringt durch das Loch 42 in der Kegelspitze 41 ins Innere der Testeinrichtung ein. Ein darin angeordnetes Vlies nimmt die Flüssigkeit solange auf, bis dieses gesättigt ist. Ein auf diesem Vlies montierter Teststreifen wird durch die Flüssigkeit aktiviert und kann anschließend ausgewertet werden. Das Testergebnis ist durch ein Fenster oder durch eine transparente Gestaltung des Endes der Testeinrichtung abzulesen. Im Rahmen der Erfindung können Testeinrichtungen eingesetzt werden, bei denen andere Ausgestaltungen für das in Berührungbringen des Teststreifens (oder der Testsubstanz) mit der Probenmischung realisiert werden. Alternativ zu der vorstehend beschriebenen Ausführungsform kann beispielsweise die Patrone ohne Einbuchtung im Bereich des Bodens ausgebildet sein. Dabei wird eine Öffnung am Boden mit einer zerstörbaren Folie versehen. Die Probenmischung wird nach Zerstörung der Folie zu dem Teststreifen geleitet, an dem gegebenenfalls eine Reaktion ablesbar ist. Die vorstehend genannte Folie ist vorzugsweise aus Aluminium oder aus einem mehrschichtigen Verbundmaterial, das vorzugsweise Aluminium und Kunststoffschichten aufweist.

Vorzugsweise werden die drei für den Benutzer sichtbaren Komponenten, die Aufnahmeeinrichtung, das Gehäuse und die Testeinrichtung durch eine unterschiedliche Farbgebung gekennzeichnet. Die Komponenten der erfindungsgemäßen Vorrichtung werden vorzugsweise als Kit dem Benutzer zur Verfügung gestellt. Beispielsweise können die hier genannten Komponenten in einer Verpackung, wie einer Blisterverpackung getrennt voneinander und gebrauchsfertig bereitgestellt werden.

Im Sinne der Erfindung bedeutet
a) "unterschiedliche Farbgebung" sowohl verschiedene Farbgebung als auch die unterschiedlich starke Einfärbung der jeweiligen Einzelkomponenten mittels ausgewählter Einzelfarbe,
b) Kit eine Packung für die Herstellung einer gebrauchsfähigen Vorrichtung zur Aufnahme und Untersuchung von Proben.

Die erfindungsgemäße Vorrichtung hat den Vorteil, daß Laien diese ohne Gefahr benutzen können. Insbesondere wird ein Austreten von Probenmaterial und der in der Patrone vorhandenen Flüssigkeit, die toxisch und ätzend sein kann, sicher vermieden.

Weiterhin unterstützt die unterschiedliche Farbgebung den folgerichtigen Zusammenbau der Komponenten.

Außerdem ist eine hygienische Handhabbarkeit gewährleistet. Durch die integrierte Aufnahme, Aufbereitung und Auftragung der Probe ist ein definiertes Verhältnis von Proben- und Reagenzienmenge sichergestellt.

Vorzugsweise werden die Komponenten aus jeweils geeignetem Kunststoff hergestellt. Bevorzugt wird ABS als Material verwendet. Dabei werden die Komponenten vorzugsweise in Spritzgußtechnik hergestellt.

Die hier beschriebenen Ausführungsformen zeigen nur Beispiele für die Realisierung der Erfindung. Dem Fachmann sind Änderungen der Ausgestaltungen in konstruktiver wie funktioneller Art möglich.

## Patentansprüche

1. Vorrichtung zur Aufnahme und Untersuchung von Proben, mit einem Gehäuse (50), in den eine Patrone (30), eine Testeinrichtung (40) und eine Aufnahmeeinrichtung (10) angeordnet sind, wobei eine mit der Aufnahmeeinrichtung (10) eingebrachte Probe mit einem Inhalt der Patrone (30) vermischbar ist und die Testeinrichtung (40) zur Auswertung der Probenmischung dient, wobei die Testeinrichtung (40) in dem Gehäuse (50) verschiebbar angeordnet ist.

2. Vorrichtung nach Anspruch 1 wobei die Aufnahmeeinrichtung (10) in ein offenes Ende des Gehäuses (50) einführbar ist.

3. Vorrichtung zur Aufnahme und Untersuchung von Proben, mit einem Gehäuse (50), in dem eine Patrone (30), eine Testeinrichtung (40) und eine Aufnahmeeinrichtung (10) angeordnet sind, wobei eine mit der Aufnahmeeinrichtung (10) eingebrachte Probe mit einem Inhalt der Patrone (30) vermischbar ist und die Testeinrichtung (40) zur Auswertung der Probenmischung dient, wobei die Aufnahmeeinrichtung (10) in ein offenes Ende des Gehäuses (50) einführbar ist und wobei die Aufnahmeeinrichtung (10) in Reihe mit der Patrone (30) angeordnet ist.

4. Vorrichtung nach Anspruch 1, 2 oder 3 wobei die Patrone (30) innerhalb des Gehäuses (50) verschiebbar ist.

5. Vorrichtung nach einem der vorstehenden Ansprüche, wobei die Aufnahmeeinrichtung (10) ein Steilgewinde (18) aufweist, das mit einem entsprechenden Innengewinde (53) im Gehäuse (51) zusammenwirkt.

6. Vorrichtung nach einem der vorstehenden Ansprüche, wobei die Aufnahmeeinrichtung (10) am probenseitigen Ende eine Probenkammer (20) aufweist.

7. Vorrichtung nach Anspruch 6, wobei die Aufnahmeeinrichtung (10) aus zwei Halbröhren (11, 12) besteht, die vorzugsweise an griffseitigen Ende durch ein Gelenk, vorzugsweise ein Filmscharnier, oder einen elastischen Steg (13) miteinander verbunden sind.

8. Vorrichtung nach Anspruch 6 oder 7, wobei mindestens eine der beiden Halbröhren (11, 12) am probenseitigen Ende eine erste und eine zweite Scheibe (21, 22) aufweist, die die Probenkammer (20) begrenzen.

9. Vorrichtung nach Anspruch 8, wobei die probenseitige Scheibe (21) ein Sieb (26) aufweist, in der Scheibe vorzugsweise eine Mulde (25) ausgebildet ist und vorzugsweise am Rand ein konusförmiger Ring (24) aufgesetzt ist, wobei die Scheibe vorzugsweise über Sollbruchstellen (23) an der Aufnahmeeinrichtung (10) befestigt ist.

10. Vorrichtung nach einem der Ansprüche 1 bis 9, wobei die Patrone (30) an einem Ende eine Öffnung (32) aufweist, die von einer Kugel (33) verschließbar ist

11. Vorrichtung nach einem der Ansprüche 1 bis 10, wobei die Patrone (30) einen Boden (34) aufweist, der perforierbar ist.

12. Vorrichtung nach Anspruch 10 oder 11, wobei die Patrone (30) zylinderförmig ausgebildet ist und an einem Ende einen Kegelstumpf (31) aufweist, der mit dem konusförmigen Ring (24) an dem Sieb (26) der Aufnahmeeinrichtung (10) in Eingriff bringbar ist, wobei die Kugel (33) mit der Mulde (25) in Berührung kommt.

13. Vorrichtung nach einem der Ansprüche 1 bis 12, wobei die Testeinrichtung die Form eines Zylinders hat, der patronenseitig eine Verjüngung in Form eines Kegelstumpfes (41) aufweist, an dessen Spitze vorzugsweise eine Öffnung (42) vorgesehen ist.

14. Vorrichtung nach einem der Ansprüche 1 bis 13, wobei die Testeinrichtung (40) mit den Inhalt der Patrone (30) in Berührung bringbar ist, wenn die Aufnahmeeinrichtung (10) im Gehäuse angeordnet ist.

15. Packung für die Herstellung einer gebrauchsfähigen Vorrichtung zur Aufnahme und Untersuchung von Proben, umfassend
a) eine Aufnahmeeinrichtung (10) zur Aufnahme einer Probe,
b) ein Gehäuse (50) mit einer darin angeordneten Patrone (30), und
c) eine Testeinrichtung (40),
wobei die Aufnahmeeinrichtung (10) und die Testeinrichtung (40) in das Gehäuse (50) einführbar sind.

16. Packung nach Anspruch 15, wobei die Testeinrichtung (40) nach ihrer Einführung in das Gehäuse (50) zusätzlich verschiebbar ist.

## Claims

1. A device for taking and examining samples comprising
a housing (50) in which a cartridge (30), a testing device (40) and a sample-taking device (10) are arranged, wherein a sample introduced with the sample-taking device (10) is miscible with a content of the cartridge (30) and the testing device (40) serves for evaluation of the sample mixture, wherein the testing device (40) is arranged displaceably in the housing (50).

2. A device according to claim 1 wherein the sample-taking device (10) can be introduced into an open end of the housing (50).

3. A device for taking and examining samples comprising
a housing (50) in which a cartridge (30), a testing device (40) and a sample-taking device (10) are arranged, wherein a sample introduced with the sample-taking device (10) is miscible with a content of the cartridge (30) and the testing device (40) serves for evaluation of the sample mixture, wherein the sample-taking device (10) can be introduced into an open end of the housing (50) and wherein the sample-taking device (10) is arranged in series with the cartridge (30).

4. A device according to claim 1, claim 2 or claim 3 wherein the cartridge (30) is displaceable within the housing (50).

5. A device according to one of the preceding claims wherein the sample-taking device (10) has a coarse screwthread (18) co-operating with a corresponding female screwthread (53) in the housing (50).

6. A device according to one of the preceding claims wherein the sample-taking device (10) has a sample chamber (20) at the end towards the sample.

7. A device according to claim 6 wherein the sample-taking device (10) comprises two half-tube portions (11, 12) which are preferably connected together at the handle end by a hinge, preferably a film hinge, or an elastic bar (13).

8. A device according to claim 6 or claim 7 wherein at least one of the two half-tube portions (11) at the end towards the sample has a first and a second disc (21, 22) which delimit the sample chamber (20).

9. A device according to claim 8 wherein the disc (21) at the sample end has a sieve (26), preferably a depression (25) is provided in the disc and a conical ring (24) is preferably fitted on at the edge, wherein the disc is preferably fixed by way of desired-rupture locations (23) to the sample-taking device (10).

10. A device according to one of claims 1 to 9 wherein at one end the cartridge (23) has an opening (32) which is closable by a ball (33).

11. A device according to one of claims 1 to 10 wherein the cartridge (30) has an end portion (34) which is perforatable.

12. A device according to claim 10 or claim 11 wherein the cartridge (30) is of a cylindrical configuration and at one end has a frustoconical portion (31) which can be brought into engagement with the conical ring (24) on the sieve (26) of the sample-taking device (10), the ball (33) coming into contact with the depression (25).

13. A device according to one of claims 1 to 12 wherein the testing device is in the form of a cylinder which at the cartridge end has a taper in the form of a truncated cone (41), preferably with an opening (42) provided at the tip thereof.

14. A device according to one of claims 1 to 13 wherein the testing device (40) can be brought into contact with the content of the cartridge (30) when the sample-taking device (10) is arranged in the housing.

15. A pack for the production of a usable device for taking and examining samples, including
a) a sample-taking device (10) for taking a sample,
b) a housing (50) with a cartridge (30) arranged therein, and
c) a testing device (40),
wherein the sample-taking device (10) and the testing device (40) can be introduced into the housing (50).

16. A pack according to claim 15 wherein after the insertion thereof into the housing (50) the testing device (40) is additionally displaceable.

## Revendications

1. Dispositif pour le prélèvement et l'examen d'échantillons, comprenant un boîtier (50), dans lequel sont agencées une cartouche (30), une unité de test (40) et une unité de prélèvement (10), un échantillon introduit au moyen de l'unité de prélèvement (10) pouvant être mélangé au contenu de la cartouche (30) et l'unité de test (40) étant destinée à analyser le mélange d'échantillon, dans lequel dispositif l'unité de test (40) est agencée de manière déplaçable dans le boîtier (50).

2. Dispositif selon la revendication 1, dans lequel l'unité de prélèvement (10) peut être engagée dans une extrémité ouverte du boîtier (50).

3. Dispositif pour le prélèvement et l'examen d'échantillons, comprenant un boîtier (50), dans lequel sont agencées une cartouche (30), une unité de test (40) et une unité de prélèvement (10), un échantillon introduit au moyen de l'unité de prélèvement (10) pouvant être mélangé au contenu de la cartouche (30) et l'unité de test (40) étant destinée à analyser le mélange d'échantillon, dans lequel dispositif l'unité de prélèvement (10) peut être engagée dans une extrémité ouverte du boîtier (50) et l'unité de prélèvement (10) est agencée en série avec la cartouche (30).

4. Dispositif selon la revendication 1, 2 ou 3, dans lequel la cartouche (30) est déplaçable à l'intérieur du boîtier (50).

5. Dispositif selon l'une quelconque des revendications précédentes, dans lequel l'unité de prélèvement (10) est munie d'un filet à pas rapide (18), qui coopère avec un taraudage (53) correspondant dans le boîtier (50).

6. Dispositif selon l'une quelconque des revendications précédentes, dans lequel l'unité de prélèvement (10), au niveau de son extrémité du côté échantillon, comporte une chambre pour échantillon (20).

7. Dispositif selon la revendication 6, dans lequel l'unité de prélèvement (10) est formée par deux demi-tubes (11, 12) qui sont assemblés l'un à l'autre, de préférence au niveau de l'extrémité du côté de manipulation par l'intermédiaire d'une articulation, de préférence un film formant charnière, ou par une barrette (13) élastique.

8. Dispositif selon la revendication 6 ou 7, dans lequel au moins un des deux demi-tubes (11, 12), au niveau de l'extrémité du côté échantillon, comporte un premier et un deuxième disque (21, 22), qui délimitent la chambre pour échantillon (20).

9. Dispositif selon la revendication 8, dans lequel le disque (21) du côté échantillon comporte un crible (26), un creux (25) est réalisé de préférence dans le disque (21) et une bague conique (24) est emmanchée de préférence sur le bord, le disque (21) étant fixé de préférence par des zones de rupture théorique (23) à l'unité de prélèvement (10).

10. Dispositif selon l'une quelconque des revendications 1 à 9, dans lequel la cartouche (30), au niveau d'une extrémité, comporte un orifice (32), qui est obturable par une bille (33).

11. Dispositif selon l'une quelconque des revendications 1 à 10, dans lequel la cartouche (30) comporte un fond (34) qui peut être transpercé.

12. Dispositif selon la revendication 10 ou 11, dans lequel la cartouche (30) est conçue en forme de cylindre avec une extrémité tronconique (31), qui peut être amenée en prise avec la bague conique (24) au niveau du crible (26) de l'unité de prélèvement (10), la bille (33) entrant en contact avec le creux (25).

13. Dispositif selon l'une quelconque des revendications 1 à 12, dans lequel l'unité de test (40) a la forme d'un cylindre qui, au niveau de l'extrémité du côté cartouche, comporte un rétrécissement en forme de cône tronqué (41), sur l'extrémité duquel est prévu de préférence un orifice (42).

14. Dispositif selon l'une quelconque des revendications 1 à 13, dans lequel l'unité de test (40) peut être amené en contact avec le contenu de la cartouche (30), lorsque l'unité de prélèvement (10) est agencée dans le boîtier.

15. Ensemble pour la réalisation d'un dispositif, apte à l'emploi, pour le prélèvement et l'examen d'échantillons, comprenant
a) une unité de prélèvement (10) pour le prélèvement d'un échantillon,
b) un boîtier (50), dans lequel est agencée une cartouche (30), et
c) une unité de test (40),
dans lequel l'unité de prélèvement (10) et l'unité de test (40) peuvent être introduites dans le boîtier (50).

16. Ensemble selon la revendication 15, dans lequel l'unité de test (40), une fois insérée dans le boîtier (50), peut en plus être déplacée.
